Europäisches Patentamt

European Patent Office

Office européen des brevets

(11) Publication number: **0 103 546**
**B1**

(12) **EUROPEAN PATENT SPECIFICATION**

(45) Date of publication of patent specification: **04.05.88**

(51) Int. Cl.⁴: **A 61 F 2/24, B 25 B 11/00**

(21) Application number: **83830164.6**

(22) Date of filing: **08.08.83**

(54) Surgical instrument for implanting prosthetic heart valves or the like.

(30) Priority: **09.08.82 IT 4896782**

(43) Date of publication of application:
**21.03.84 Bulletin 84/12**

(45) Publication of the grant of the patent:
**04.05.88 Bulletin 88/18**

(84) Designated Contracting States:
**BE DE FR GB NL**

(56) References cited:
**IT-A-5 058 679**
**US-A-3 409 013**
**US-A-4 294 141**

**MECHANICAL ENGINEERING, vol. 89, no. 1, January 1967, page 48 "Heart valve sans suture"**

(73) Proprietor: **Iorio, Domenico**
**Via S. Francesco d'Assisi, 203**
**I-81024 Maddaloni (Caserta) (IT)**

(72) Inventor: **Iorio, Domenico**
**Via S. Francesco d'Assisi, 203**
**I-81024 Maddaloni (Caserta) (IT)**

(74) Representative: **Massari, Marcello**
**Studio M. Massari S.r.l. 23, Via Fontanella Borghese**
**I-00186 Roma (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention refers to an instrument to be employed in surgery and intended to render more easy and secure the implant of bioprothesis in human organs and more particularly the implant of a valvular bioprothesis in mitralic position, and it is in connection with this intervention of high "open heart" surgery that the invention is disclosed and illustrated.

More precisely, the invention concerns an instrument that is an improvement of the instrument subject matter of Italian patent application No. 50586A/79 filed on 16th October 1979, of the same applicant.

As it is known to cardiologists and heart surgeons, serious mitralic alterations are now surgically treated by substituting the mitralic valve with a valvular bioprothesis.

This valvular bioprothesis comprises a base or support of a biocompatible synthetic material, as the material sold under the Trade Mark "TEFLON" and three "tabs" of animal origin which constitute the moving parts of the valvular bioprothesis.

The base consists of an annular lower part and three "peaks" circumferentially spaced by 120° and separated by three "saddles" which form together an up-and-down profile to the edges of which the tabs of animal origin are connected.

To fix this bioprothesis in place in the patient's heart, a number of threads are utilized, one end of which is anchored to the mitralic annulus of the patient that has previously been deprived of the altered mitralic valve. These threads are inserted in a flange provided at the lower annular part of the bioprothesis base and the bioprothesis is caused to slide along the threads until the flange sets against the mitralic annulus to which the same is then sewed by means of the same threads.

This surgical technique has the serious drawback that, during the sliding movement toward the mitralic annulus, one or more threads can get entangled in the bioprothesis support, that has a rough surface, and consequently either a distortion occurs of the bioprothesis, when the end of the entangled threads are tied, or a faulty connection of the bioprothesis is obtained with the dramatic consequence that the intervention must be repeated.

At present, to avoid this dangerous occurrence, the connection of the bioprothesis to the annulus is inspected from the inside by means of a dentist's mirror. It will be evident that such inspection, under the conditions in which this kind of intervention is carried out, besides being very difficult and inaccurate, unduly protracts the duration of the intervention.

The object of the above mentioned invention of the applicant was to provide an instrument adapted to support the bioprothesis and having expandable means that, in the not-expanded state, allows the bioprothesis to be placed on the instrument supporting head and removed therefrom, while in the expanded state, it keeps the threads spaced apart from the bioprothesis base, thus avoiding that the threads may get entangled therein.

This previous instrument comprises a rigid tubular body having a fixed head adjacent one end thereof and a small spherical ball of a resilient material fitted on this end, that can be inflated or deflated through the opposite end of the body. To this purpose, the opposite end is provided with a one-way valve and can cooperate with a device adapted to supply air under a slight pressure.

This instrument, although quite satisfactory, has some drawbacks.

First of all the fixed head supporting the bioprothesis cannot fit the different dimensions of the bioprotheses which, according to the dimension of the heart to which they must be implanted, widely vary both in diameter and height. Particularly the height or axial dimension of the bioprothesis varies considerably as between a minimum of 11 mm and a maximum of 26 mm.

This wide variation of the bioprothesis height results from the fact that the same is proportional to the base diameter which, in turn, is due to the diameter of the mitralic annulus of the patient's heart.

Besides, the spherical shape of the expandable ball did not prove to be optimal, since the outer spherical cap thereof not only is unuseful to its function but it may also constitute an obstacle to the introduction of the bioprothesis in place.

Accordingly, it is an object of the invention to provide an instrument improved in comparision with the object of the previous application mentioned above, wherein the position of the bioprothesis supporting head can be varied with respect to the adjacent end of the rigid body according to the axial dimension of the bioprothesis to be implanted, so that the latter and the expandable member fitted to the adjacent end are in the reciprocally optimal positions.

A further object of the invention is to provide an improved instrument having an expandable member having a shape different from the spherical shape. More precisely this member has the truncated shape of a molar tooth that proved to be optimal to the function this member has to perform. This shape can be easily obtained by either forming the expandable member in two parts or in one part only.

The invention will now be described in more details with reference to the attached drawings, in which:

Fig. 1 shows a section of the instrument of the invention with the expandable member in the deflated state;

Fig. 2 is a similar section showing a bioprothesis placed on the instrument, and the particular shape, Fig. 3 is a fragmentary section showing the instrument adjusted for implanting a bioprothesis of a certain height;

Fig. 4 is a similar section showing the instrument adjusted for implanting a much higher bioprothesis than the bioprothesis shown in Fig. 3; and

Fig. 5 shows a perspective view of the base of a valvular bioprothesis to be implanted.

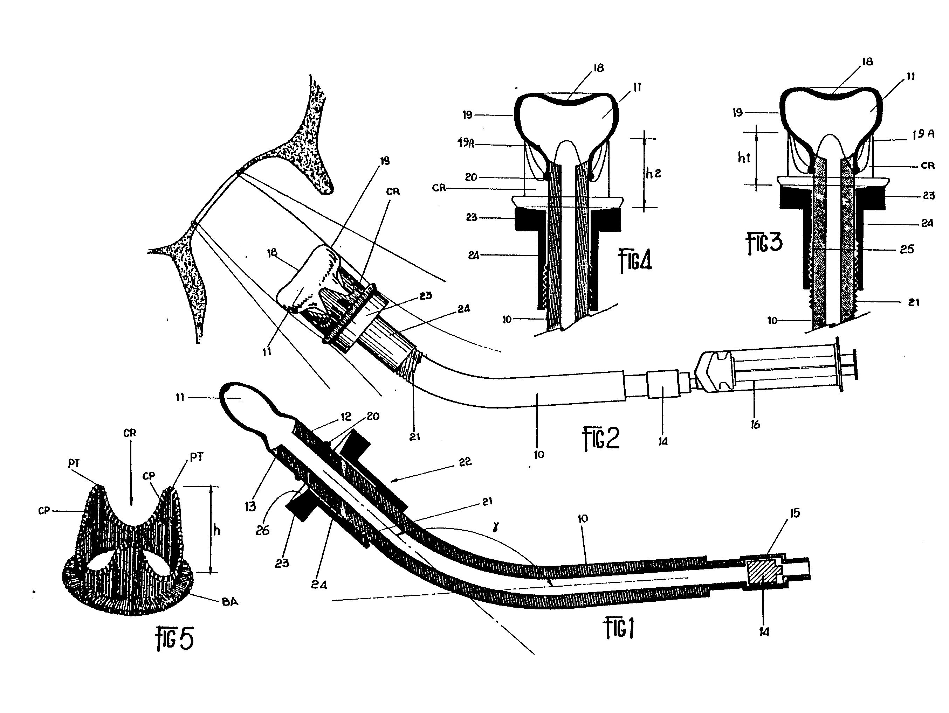

Particularly referring to Figs. 1 to 4, the instrument of the invention comprises: a tubular body 10, an expandable supporting member 11 the mouth 12 of which is sealingly fitted on one end of body 10, referred to by 13; a supporting member 22 for the bioprothesis cooperating with body 10; a valve 14 placed in the end 15 of body 10 opposite to end 13 of the expandable member 11; and a device 16, consisting of a syringe (Fig. 2) adapted to be fitted to end 15 for inflating member 11 through the bore of body 10.

Expandable member 11 is generally of the shape of a molar tooth crown of circular cross-section having a flat or slightly concave upper portion 18 and a wall or skirt 19 depending therefrom merging with mouth 12 sealingly fitted over end 13 of body 10 that is provided, to this purpose, with an annular projection 20.

As easily viewed in Figs. 2 and 3, a length 21 of body 10 is externally threaded at a little distance from end 13.

Unlike the known instrument, in the instrument of the invention, support 22 for the bioprothesis is formed separately from body 10 and comprises an annular head 23 having an integrally formed sleeve referred to by 24, having an inner diameter greater than the outer diameter of body 10. Furthermore, a length 25 of inner surface of sleeve 24 is also threaded so that it can be screwed on threaded length 21 of body 10.

Accordingly, support 22 is assembled on body 10 by fitting the latter into sleeve 24 and screwing it onto threaded length 21 whereby the position of annular head 23 can be varied with respect to end 13 of body 10 and the same depends on the entity of the screwing of head 22 onto length 21.

The pitch of matching threads 21 and 25 is very short and consequently the engagement of the two pieces together is irreversible.

By this construction it is possible to adjust the axial position of supporting head 23 on body 10 with respect to end 13. Therefore the distance between upper face 26 of head 23 and the base of skirt 19 of expandable member 11 is identical with the height "h" of the bioprothesis measured between base BA of the flange and point PT of cusps CP, i.e. to the axial length of the bioprothesis to be implanted, this height being "h1" in the case of Fig. 3 and "h2" in the case of Fig. 4, with h2>h1.

In this way, the valvular prothesis carried by annular head 23 is kept exactly in place by expandable member 11, whatever its height may be.

As regards the utilization of the above described instrument, after the insertion of a number of threads (Fig. 2) in the mitralic annulus of heart of the patient and the insertion of some of them in the annular lower part BA of support CR with member 11 in the deflated condition, end 13 of tubular body 10 of the instrument is fitted in the bioprothesis until lower part BA thereof is placed against upper face 26 of head 23 that will be in an axially inner position.

The point of syringe 16 is then introduced in the bore 15A of body 10 (Fig. 2) so that air under a slight pressure is injected into expandable member 11 to inflate it. Thereupon supporting head 23 is caused to move toward end 13 by screwing sleeve 24 on thread 21 in order to lightly clamp the bioprothesis between annular head 23 and skirt 19, as it is clearly illustrated in Figs. 3 and 4.

As clearly illustrated in Fig. 2, the diameter of lateral skirt 19 is substantially identical with the diameter of annular head 23, when member 11 is inflated, accordingly sewing threads are kept in tension, clear of support cusps CP and base flange BA of support CR while the bioprothesis is guided to place against the mitralic annulus along threads.

Furthermore, since lower part 19A of skirt 19 has a smaller diameter than upper part 19, the points PT of cusps CP are slightly retracted, whereby overhanging 19 of member 11 is radially projecting with respect thereto, thus avoiding that these points PT can hook sewing threads.

Once support CR is inserted in place, i.e. against the mitralic annulus, wherein the valving action is performed by the flaps of animal origin connected to the support, which flaps are not shown, member 11 is deflated by acting on the syringe 16, thus causing the air in member 11 to flow out through tubular body 10.

End 13 of the instrument can now be easily taken out of the bioprothesis, that is then sewed to place by means of threads.

It is important to note that the bent shape of body 10 is very useful as it allows an easier and better introduction of the bioprothesis, particularly in the case wherein the patient undergoing the intervention is affected by an advanced calcification of the mitralic annulus that does not allow the annulus to be exposed at the level of the atrium in order to be easily reached by the surgeon.

In connection therewith it is convenient to point out that angle γ of the bending of the two lengths of body 10 is suitably between 120° and 150°, particularly an angle of 135° proved to be optimal.

As regards the material of which the instrument of the invention is made, it will be a not expensive, biocompatible plastic material, as PVC. This will allow a disposable instrument to be supplied together with the valvular bioprothesis, that is not going to be reutilized, thus avoiding any problem of sterilization.

To the persons skilled in the art it will be evident that the invention is not limited to the shape thereof here described and illustrated and that the same can be made in a different way, provided that the concept is respected of providing an instrument suitable for supporting a valvular bioprothesis for a rapid and secure implant thereof and provided with an end having variable radial dimensions between a reduced dimension allowing an easy placing and removal of the bioprothesis and a maximum dimension, efficient in keeping the sewing threads in tension and clear of the bioprothesis support.

In view of what stated above, all instruments will be in the invention scope, having at one end expandable means, of mechanical, hydraulic or other kind, besides the pneumatic means herein described and illustrated.

## Claims

1. Instrument intended to be used in surgery for implanting a valvular bioprothesis in mitralic position, in the mitralic annulus of a patient's heart, which bioprothesis comprises: a support (CR) of a synthetic material, comprising an annular base (BA) and three cusps (CP) with pointed tops (PT) integral herewith, and three flaps or limbs of a valve of animal origin operatively supported by the support (CR), the bioprothesis being intended to be functionally united to the mitralic annulus, deprived of its own mitralic valve, by means of sewing threads previously inserted both in the annulus edge and in a flange integral with the base (BA), which instrument comprises: a tubular body (10) having a first (13) and a second end (15); a supporting means (23) for the bioprothesis carried on the tubular body; and expandable member (11) having a circular cross section mounted on the first end (13) and adapted to assume a radially retracted condition when the bioprothesis has to be fitted onto or removed from the supporting means (23), and a radially expanded condition wherein the member (11) has a diameter at least equal to the diameter of the supporting means (23), so that the member (11) engages and keeps away the sewing threads to avoid entanglement of the threads in any part of the bioprothesis and controlling means (16) for the member (11) mounted on the second end (15) of the body (10) for controlling the radial condition of the member (11), characterised in that the supporting means (23) is movable to vary its axial position relative to the first end (13) in accordance with the axial dimension of the bioprothesis and the expandable member (11) has a flat or slightly concave upper portion (18) and a skirt (19) depending therefrom.

2. Instrument according to claim 1, wherein the tubular body (10) has an externally threaded length (21) adjacent to the first end (13), and the supporting means comprises an annular head (23) presenting an upper face (26) for receiving the bioprothesis base (BA) and an integral sleeve (24) provided with an internal thread (25) matching thread (21) of the body (10), so that the axial position of supporting head with respect to the first end (13) can be varied by screwing the sleeve (24) on threaded length (21).

3. Instrument according to claim 1, wherein the body (10) has a bent shape and forms an angle (γ) comprised between 120° and 150° in order to facilitate the placement of the bioprothesis.

4. Instrument according to claim 3, wherein the bending angle is 135°.

5. Instrument according to claim 1, wherein said member (11) comprises an inflatable member (11) including a flat upper portion (18) and a skirt depending therefrom (19), the mouth (12) of which is sealingly fitted on the first end (13) of the body (10) that holds the supporting means (22) for the bioprothesis, which member (11), when inflated, is shaped as a molar tooth having such a diameter to engage with the sewing threads.

## Patentansprüche

1. Chirurgisches Instrument zum Anlegen einer Herzklappenbioprothese in Mitrallage, nämlich in den Mitralanulus des Herzens eines Patientes, welche Herzklappenbioprothese enthält: ein aus einer ringförmigen, mit drei Spitzen (CP) einstückigen Grundlage (BA) bestehendes Tragkissen (CR) aus Kunststoff; und drei Klappen eines Ventils tierischer Herkunft, die mit dem Tragkissen (CR) eingreifen, wobei die Herzklappenbioprothese mit dem Mitralanulus ohne seine Mitralklappe über Fäden eingreift, die in den Mitralanulusrand sowie einen mit der Grundlage (BA) einstückigen Flansch vorher eingefädelt werden, welches Instrument enthält: einen rohrförmigen Körper (10) mit einem ersten (13) und einem zweiten Ende (15); einen Auflageteil (23) für die von dem rohrförmigen Körper abgestützte Herzklappenbioprothese; einen ausdehnbaren, einen kreisförmigen Querschnitt aufweisenden Teil (11), der auf dem ersten Ende (13) angebracht ist und einen radiall zusammengezogen Zustand dann einnehmen kann, wenn die Herzklappenbioprothese auf den bzw. von dem Auflageteil (23) anzulegen bzw. abzunehmen ist, sowie einen radiall ausgedehnten Zustand einnehmen kann, bei dem der Teil (11) einen zumindest gleichen Durchmesser wie den Durchmesser des Auflageteiles (23) aufweist sowie die Fäden eingreift und voneinander getrennt hält, so daß sich die Fäden mit der Herzklappenbioprothese nicht verfangen können; und einen auf dem zweiten Ende (15) des Körpers (10) angebrachten Steuerteil (16) zur Steuerung des radiallen Zustandes des Teiles (11), dadurch gekennzeichnet, daß der Auflageteil (23) zur Änderung seiner Axiallage bezüglich des ersten Endes (13) je nach dem Axialmass der Herzklappenbioprothese bewegbar ist, und daß der ausdehnbarer Teil (11) einen oberen, flachen oder ein wenig konkaven Abschnitt (18) und eine mit diesem Abschnitt verbundene Seitenklappe (19) aufweist.

2. Instrument nach Anspruch 1, dadurch gekennzeichnet, daß der rohrförmige Körper (10) einen mit einem Aussengewinde versehenen, dem ersten Ende (13) anliegenden Abschnitt (21) aufweist, und daß der Auflageteil einen ringförmigen, mit einer Oberfläche (26) zur Aufnahme der Grundlage (BA) der Herzklappenbioprothese versehenen Kopf (23) und eine mit einem das Aussengewinde (21) des Körpers (10) eingreifenden Innengewinde (25) versehene Hülse (24) aufweist, so daß die Axiallage des Auflagekopfes bezüglich des ersten Endes (13) unter Aufschrauben der Hülse (24) auf das Aussengewinde .(21) verschoben werden kann.

3. Instrument nach Anspruch 1, dadurch gekennzeichnet, daß der Körper (10) eine Krümmung derart aufweist, daß ein Winkel vom 120° zum 150° zum Erleichtern des Anlegens der Herzklappenbioprothese gebildet wird.

4. Instrument nach Anspruch 3, dadurch gekennzeichnet, daß der Krümmungswinkel 135° beträgt.

5. Instrument nach Anspruch 1, dadurch gekennzeichnet, daß der Teil (11) ausdehnbar ist und einen flachen, oberen Abschnitt (18) sowie eine mit diesem Abschnitt verbundene Seitenklappe (19) aufweist, deren Einmündung (12) dicht auf dem ersten Ende (13) des Körpers (10) aufgebracht ist, das den Auflageteil (22) der Herzklappenbioprothese festhält, wobei der Teil (11), wenn er in ausgedehntem Zustand ist, als einen Backenzahn mit einem solchen Durchmesser zum Eingreifen der Fäden ausgebildet ist.

**Revendications**

1. Appareil chirurgical pour l'implantation de bioprothèses de valvule en position mitrale dans l'anneau mitrale du coeur d'un patient où cette bioprothèse comprend: un support (CR) d'un matériel synthétique comprenant une base annulaire (BA) et trois cuspides (CP), ayant des sommités pointues (PT), intégralement formées avec dite base et trois limbes d'une valvule d'origine animale portés fonctionnellement par ledit support (CR), ladite bioprothèse étant destinée à être fonctionnellement unie à l'anneau mitrale, privé de sa valvule mitrale, par des fils de ligature préalablement introduits soit dans le bord de l'anneau soit dans un'aile formée intégralement avec la base (BA); ledit appareil comprenant:

un corps tubulaire (10) ayant une première (13) et une deuxième (15) extrémité; des moyens de support (23) de la bioprothèse portés par le corps tubulaire; un élément enflatable (11) ayant une section transversale circulaire, porté par la première extrémité (13) et apte à prendre une condition retirée selon la direction radiale quand la bioprothèse doit être introduite dans les moyens de support ou enlevée de ces derniers et une condition dilatée où ledit element (11) a une diamètre au moins égale à la diamètre des moyens de support (23), de sorte que l'élément (11) engage et eloigne les fils de ligature, pour eviter qu'ils s'accrochent à n'importe quelle partie de la bioprothèse, et des moyens de contrôle pour l'élément (11) supportés sur ladite deuxième extrémité du corps (10) pour determiner la condition dans la direction radiale de l'élément (11), caractérisé en ce que lesdits moyens de support (23) sont mobiles de façon à changer leur position axiale par rapport à ladite première extremité (13), conformement à la dimension axiale de la bioprothèse et ledit élément enflatable (11) presente une partie supérieure (18) plate ou légèrement concave et un pan (19) qui descend de cette partie.

2. Appareil selon la revendication 1, caractérisé en ce que ledit corps tubulaire (10) presente une partie taraudée (21) auprès de ladite première extrémité (13) et lesdits moyens de support comprennent une tête annulaire (23), qui presente une surface supérieure (26) pour recevoir la base (BA) de la bioprothèse, et un manchon (24) formé integralement muni d'un taraudage intérieur (25) qui engage le taraudage (21) du corps 10, de façon que la position axiale de la tête de support à l'égard de la première extrémité (13) peut être changée en vissant le manchon (24) sur la partie taraudée (21).

3. Appareil selon la revendication 1, caractérisé en ce que le corps (10) presente une forme courbée formant un angle (γ) compris entre 120° et 150° dans le but de faciliter l'installation de la bioprothèse.

4. Appareil selon la revendication 3, caracterisé en ce que l'angle de courbure est de 135°.

5. Appareil selon la revendication 1, caracterisé en ce que ledit l'element (11) est constitué par un element enflatable (11) qui comprend une partie supérieure plate (18) et un pan (19) qui descend de cette partie, dont la bouche (12) est enfilée d'une façon étanche sur la première extrémité (13) du corps (10) qui porte les moyens de support (22) de la bioprothèse, lequel élement, quand il est enflaté, presente la forme d'une dent molaire ayant une diamètre telle qu'il engage les fils de ligature.

FIG1

FIG2

FIG3

FIG4

FIG5

0 103 546